(19) **Europäisches Patentamt / European Patent Office / Office européen des brevets**

(11) **EP 2 064 171 B2**

(12) **NOUVEAU FASCICULE DE BREVET EUROPEEN**
Après la procédure d'opposition

(45) Date de publication et mention de la décision concernant l'opposition:
**02.06.2021 Bulletin 2021/22**

(45) Mention de la délivrance du brevet:
**07.03.2012 Bulletin 2012/10**

(21) Numéro de dépôt: **07823502.5**

(22) Date de dépôt: **13.09.2007**

(51) Int Cl.:
**C07C 209/48** (2006.01)　　**C07C 211/12** (2006.01)

(86) Numéro de dépôt international:
**PCT/FR2007/001476**

(87) Numéro de publication internationale:
**WO 2008/034964 (27.03.2008 Gazette 2008/13)**

(54) **PROCEDE DE FABRICATION D'AMINES PAR HYDROGENATION DE COMPOSES NITRILES**

VERFAHREN ZUR HERSTELLUNG VON AMINEN DURCH HYDRIERUNG VON NITRILVERBINDUNGEN

METHOD FOR PRODUCING AMINES BY THE HYDROGENATION OF NITRILE COMPOUNDS

(84) Etats contractants désignés:
**AT BE BG CH CY CZ DE DK EE ES FI FR GB GR HU IE IS IT LI LT LU LV MC MT NL PL PT RO SE SI SK TR**

(30) Priorité: **19.09.2006 FR 0608172**

(43) Date de publication de la demande:
**03.06.2009 Bulletin 2009/23**

(73) Titulaire: **BASF SE**
**67056 Ludwigshafen (DE)**

(72) Inventeurs:
- **ROCCATI, Philippe**
  **01800 Charnoz Sur Ain (FR)**
- **LETOURNEUR, Didier**
  **68170 Rixheim (FR)**

- **DENIS, Philippe**
  **69150 Decines (FR)**

(74) Mandataire: **BASF IP Association**
**BASF SE**
**GBI-C006**
**67056 Ludwigshafen (DE)**

(56) Documents cités:
| | |
|---|---|
| WO-A-00/37424 | WO-A-95/17959 |
| WO-A-98/43940 | WO-A1-98/43940 |
| FR-A1- 2 722 784 | US-A- 3 972 940 |
| US-A- 4 429 159 | US-B1- 6 232 488 |

- **Winnacker, Küchler: "Chemische Technik, Prozesse und Produkte.", , vol. 5, 2005, pages 242-244,**

EP 2 064 171 B2

## Description

La présente invention concerne un procédé de fabrication d'hexaméthylène diamine par hydrogénation de l'adiponitrile en présence d'un catalyseur.

Elle concerne plus particulièrement un procédé de fabrication d'hexaméthylène diamine par hydrogénation, en continu, de l'adiponitrile en présence d'un catalyseur à base d'un métal de Raney.

Un des procédés de fabrication d'hexaméthylène diamine utilisé industriellement est l'hydrogénation catalytique de l'adiponitrile.

Ainsi, l'hexaméthylène diamine qui est un intermédiaire chimique important pour la fabrication de nombreux composés ou polymères, est produite, majoritairement, par hydrogénation catalytique de l'adiponitrile.

A titre d'exemple, on peut citer les procédés d'hydrogénation de l'adiponitrile en présence d'un oxyde métallique, comme catalyseur tel que l'oxyde de fer ou de cobalt. Ces procédés sont généralement mis en œuvre à des pressions et températures élevées et souvent en présence d'ammoniac.

D'autres procédés d'hydrogénation largement exploités consistent à utiliser comme catalyseur, un système à base d'un métal de Raney, plus particulièrement de Nickel ou Cobalt de Raney, en présence d'eau et d'un composé basique. Dans ces procédés, la réaction est réalisée à des pressions et températures peu élevées et en absence d'ammoniac. Ces derniers procédés d'hydrogénation avec catalyseur à base de métal de Raney peuvent être mis en œuvre dans certains types de réacteur. En effet, comme le catalyseur est pyrophorique, les réacteurs utilisés doivent permettre un contrôle efficace de l'introduction en continu du catalyseur dans le milieu réactionnel. Il est également nécessaire de contrôler la quantité de catalyseur mise en suspension dans le milieu réactionnel et d'assurer une circulation maîtrisée et contrôlée de cette suspension.

Cependant, l'activité de ces catalyseurs peut être fortement affectée quand ils sont utilisés sous certaines conditions dans les procédés d'hydrogénation de l'adiponitrile.

Un article publié dans Chemical Engineering Science vol 47 n°9-11, 2 289-94 (1992) indique que les composés nitriles désactivent les catalyseurs à base de Nickel ou Cobalt de Raney. Pour éviter cette désactivation, il a été proposé, dans l'article publié dans Chemical Engineering Science Vol 35, 135-141 (1980) d'utiliser un réacteur avec une vitesse de circulation élevée du milieu réactionnel pour obtenir des conditions de mélanges optimales et un flux turbulent pour éviter la formation de zones présentant une concentration élevée en composés nitriles. En effet, une concentration élevée en composés nitriles induit une désactivation du catalyseur.

Tous ces procédés utilisent comme solution pour diminuer la dégradation de l'activité du catalyseur, des systèmes permettant d'avoir un mélange efficace du milieu réactionnel et donc avoir une concentration en réactifs et catalyseur identiques en tout point du réacteur.

Une telle qualité de mélange est difficile à obtenir et demande des dispositifs complexes comme ceux décrits dans la demande de brevet WO00/37424 qui concerne l'utilisation de mélangeurs statiques pour améliorer la qualité du mélange et l'homogénéité des concentrations en tout point du réacteur.

En outre, ces solutions ne peuvent s'appliquer quand la réaction d'hydrogénation est mise en œuvre, en continu, dans un réacteur fonctionnant sous un régime piston tel que les colonnes à bulles fonctionnant avec circulation d'un lit de catalyseur en suspension, les réacteurs comprenant une colonne à bulles et une tête cyclone pour séparer les gaz, appelés ci-après réacteurs de type RTC.

Un des buts de la présente invention est de proposer un procédé continu d'hydrogénation de l'adiponitrile en hexaméthylène diamine en présence d'un catalyseur à base de métal de Raney permettant de limiter la vitesse de dégradation de l'activité du catalyseur et en évitant la formation d'impuretés difficilement séparables de l'aminé formée.

A cet effet, l'invention propose un procédé continu de fabrication d'un composé comprenant au moins une fonction amine par hydrogénation d'un composé comprenant au moins une fonction nitrile caractérisé en ce qu'il consiste à :

❖ Alimenter un gaz contenant de l'hydrogène et un composé nitrile, dans un réacteur de type piston dans lequel circule un milieu réactionnel comprenant des particules en suspension de catalyseur à base de métal de Raney, une base minérale et de l'eau,
❖ soutirer en sortie du réacteur piston, une partie du milieu réactionnel contenant le composé amine après séparation du catalyseur et recycler l'autre partie dans le réacteur piston.
❖ recycler le catalyseur séparé dans le réacteur piston,
❖ alimenter dans le réacteur piston un flux de catalyseur neuf,

et en ce que, les débits d'alimentation en composés nitriles et/ou en catalyseur dans le réacteur piston sont contrôlés pour maintenir un rapport P entre le nombre de mole de composés nitriles alimentés par unité de temps et le débit massique de catalyseur dans le réacteur piston compris entre 0,02 et 0,15 mole de composés nitriles par kg de catalyseur pour un catalyseur présentant une activité initiale comprise entre $15.10^{-5}$ et $35.10^{-5}$ mole de $H_2$/g de catalyseur /s et en ce que le composé nitrile est l'adiponitrile, l'aminé synthétisée étant l'hexaméthylène diamine.

Le débit massique de catalyseur peut être obtenu par le bilan matière du dispositif utilisé ou par analyse de la concentration en catalyseur dans le réacteur piston ou dans d'autres parties de l'installation telles que dans la partie assurant le recyclage du milieu réactionnel dans le réacteur piston.

Il peut également être obtenu par mesure de la concentration en catalyseur à différents emplacements de l'installation où circule le catalyseur, tels que, par exemple, dans le flux de recyclage du catalyseur dans le réacteur piston après séparation du milieu réactionnel, et extrapolation mathématique en prenant en compte l'hydrodynamique de l'installation utilisée pour la mise en œuvre de cette réaction. Cette extrapolation mathématique ou corrélation entre la mesure de la concentration en catalyseur et le débit de catalyseur dans le réacteur piston est établie pour chaque installation par application des règles hydrodynamiques bien connues de l'état de la technique, ou par des mesures systématiques pour établir une courbe ou abaque d'étalonnage.

L'activité du catalyseur est exprimée par la quantité molaire d'hydrogène consommée lors de la réduction de l'adiponitrile en hexaméthylène diamine pour une quantité de catalyseur Nickel de Raney définie par unité de temps dans des conditions de température et de pression et un rapport KOH/masse de catalyseur déterminés pour la méthode de mesure.

L'activité initiale du catalyseur est déterminée en mettant en œuvre le mode opératoire décrit ci-dessous :

Dans un bécher de 250 ml, verser 50 ml d'eau déminéralisée et désaérée à l'argon.

A l'aide d'une spatule, prélever environ 2 grammes de bouillie de catalyseur à analyser

Introduire la prise d'essai dans le bécher et ajouter environ 100ml d'eau déminéralisée et désaérée. Agiter le milieu pour mettre en suspension le catalyseur à analyser.

Séparer l'eau du catalyseur en posant une plaque aimantée au fond du bécher.

Vider l'eau déminéralisée et désaérée.

Verser 150 ml d'eau déminéralisée dans le bécher.

Le bécher posé sur l'aimant : vider l'eau de lavage puis ajouter 50 à 100ml d'eau désaérée.

Laver le catalyseur à l'eau déminéralisée et désaérée. A la fin des lavages, le pH de l'eau doit être voisin de 7.

Remplir un pycnomètre avec le liquide surnageant du catalyseur.

Peser le pycnomètre pour définir le zéro de la balance.

Enlever le pycnomètre de la balance.

Enlever une petite quantité d'eau du picnomètre avec une pipette.

Prélever dans le bécher, le catalyseur avec la même pipette.

Introduire une quantité de catalyseur dans le picnomètre et réajuster le pycnomètre avec le liquide surnageant.

Peser le pycnomètre et lire la différence de masse (M) entre le pycnomètre rempli d'eau plus le catalyseur et le pycnomètre rempli d'eau. La masse de catalyseur $m_{catalyseur}$ est égale à 1,15*M. Réajuster la quantité de catalyseur dans le pycnomètre en répétant les opérations de pesée ci-dessus jusqu'à obtenir la quantité désirée :

La quantité de catalyseur à prélever est avantageusement égale à :

0,4000 g environ exactement de catalyseur lavé neuf, régénéré ou usé :

$(0,3960 \text{ g} \leq m_{catalyseur} \leq 0,4040 \text{ g})$

Noter la masse $m_{catalyseur}$ de catalyseur introduite dans le pycnomètre (précision : 0,0001 gramme).

Poser le réacteur propre et sec, ainsi que son support sur le trébuchet. Faire le zéro.

Transvaser le contenu du pycnomètre dans un réacteur propre et sec.

Rincer le pycnomètre avec la quantité nécessaire d'eau déminéralisée pour recueillir la totalité du catalyseur introduit dans le pycnomètre.

Eliminer le maximum d'eau en utilisant la plaque aimantée sur le fond du réacteur.

Ajouter sur le catalyseur de la potasse KOH 6.8N à l'aide de la micro seringue adaptée soit 47 $\mu$l Compléter avec de l'eau déminéralisée pour obtenir un poids de solution dans le réacteur égal à 4,66 g

Ajouter 37,8 g d'HMD pure dans le réacteur.

Installer le réacteur sur le dispositif pilote et mettre le chauffage en route (Température de consigne 80°C).

Purger 3 fois le ciel du réacteur avec de l'azote.

Mettre la réserve d'hydrogène à une pression de 50 bar.

Purger le ciel du réacteur à l'hydrogène par admission d'hydrogène et évacuation. Répéter l'opération 3 fois en maintenant ou rétablissant la pression dans la réserve d'hydrogène.

A la fin des trois purges à l'hydrogène du réacteur, pressuriser le réacteur à 20 bar en hydrogène.

Mettre l'agitation en route (température de consigne de 80°C et vitesse d'agitation à 2000 trs.min-1). Prélever environ exactement 6,00 grammes d'adiponitrile avec une seringue en verre munie de son aiguille rallongée.

Faire le zéro de la balance avec la seringue chargée et munie de son aiguille.

Introduire l'adiponitrile dans une ampoule de coulée.

Peser la seringue vide munie de son aiguille.

Noter la masse d'adiponitrile $m_{ADN}$ introduite dans l'ampoule de coulée (elle doit être comprise entre 5,70 et 6,30 grammes).

Mettre le réacteur sous pression d'hydrogène à partir de la réserve d'hydrogène et rétablir une pression de 50 bar dans la réserve. Vérifier que les pressions de réserve et de réacteur restent constantes.

Une fois la température et l'agitation atteinte, Ouvrir la vanne de l'ampoule de coulée d'abord doucement puis complètement en une seule opération pour introduire l'AdN dans le réacteur.

La pression à l'intérieur du réacteur doit monter à 25 bar.

L'alimentation en hydrogène dans le réacteur à partir de la réserve est maintenue ouverte jusqu'à ce que la courbe de pression d'hydrogène soit stable.

Pendant la durée de la réaction, la pression d'hydrogène dans la réserve est enregistrée en fonction du temps.

Quand la pression d'hydrogène dans le réacteur est stable, l'alimentation en hydrogène dans le réacteur est fermée.

Le chauffage du réacteur est coupé et le milieu réactionnel est refroidi jusqu'à une température de 45°C.

Décomprimer doucement le réacteur et arrêter l'agitation.

Purger trois fois le ciel du réacteur avec de l'azote.

Vider le contenu du réacteur dans un récipient avec rinçage à l'eau déminéralisée.

L'activité du catalyseur est déterminée par la vitesse initiale de la réaction donnée par la formule suivante :

$$V_{initiale} = \frac{4 \text{ x } m_{ADN}}{108 \text{ x } m_{catalyseur} \text{ x } 60 \text{ x } \Delta t_{initial}}$$

$\Delta t_{initial}$ est le temps en minute que mettrait l'hydrogénation si le catalyseur était renouvelé en permanence durant la réaction. Il est déterminé par l'abscisse du point d'intersection entre la pente de la courbe d'enregistrement de la pression au départ de la réaction et la pente à la fin de la réaction qui est généralement nulle. Cette courbe représente la variation de la pression d'hydrogène dans la réserve en fonction du temps. La vitesse initiale d'hydrogénation est exprimée en mole $H_2$. $g^{-1}cata.s^{-1}$.

Par contrôle de ce rapport P pour un catalyseur présentant une activité catalytique dans un domaine déterminé, la demanderesse s'est aperçu, contrairement à l'enseignement des connaissances acquises sur cette réaction, que l'hexaméthylène diamine récupérée contient une très faible proportion d'impuretés, notamment de diaminocyclohexane et que la vitesse de dégradation de l'activité du catalyseur est très faible.

En effet, il est connu que dans certaines conditions, l'adiponitrile peut réagir pour donner par hydrogénation une diamine cyclique, le diaminocyclohexane qui est difficilement séparable de l'hexaméthylène diamine. En conséquence, il est important de limiter la formation de cette diamine cyclique pour obtenir une hexaméthylène diamine qui pourra être purifiée avec un investissement et une consommation d'énergie minimum. Il est connu que cette formation d'impuretés de type diaminocyclohexane peut être limitée si le rapport P de l'adiponitrile à la masse de catalyseur est élevée. Toutefois, dans ces conditions, il est également connu que la vitesse de dégradation de l'activité du catalyseur est élevée.

La demanderesse s'est également aperçu que l'hexaméthylène diamine obtenue en respectant les caractéristiques de fonctionnement décrites ci-dessus, présente une faible concentration en impuretés qui sont détectées par l'analyse UV de l'hexaméthylène diamine, caractéristique de pureté exprimée généralement sous forme d'un indice UV (Iuv). Cet indice est obtenu par mesure de l'absorbance UV à une longueur d'onde de 275nm d'une solution aqueuse de HMD à 32,4% en poids contenue dans une cuve de longueur de 5 cm.

L'invention propose des conditions de mise en œuvre de la réaction permettant d'une part de limiter la formation d'impuretés du type diaminocyclohexane (DCH) et/ou détectables par l'analyse UV. Ces conditions correspondent à l'utilisation d'un rapport P (débit molaire de composé nitrile sur débit massique de catalyseur dans le réacteur piston) élevé en combinaison avec un catalyseur présentant une activité catalytique déterminée. Dans ces conditions et bien que le rapport P soit élevé, la vitesse de dégradation de l'activité du catalyseur reste à un niveau très faible, résultat normalement obtenu pour un rapport P bas.

Le procédé de l'invention s'applique à l'hydrogénation de l'adiponitrile en hexaméthylène diamine.

Le procédé de l'invention permet par l'utilisation d'un rapport P, débit molaire de l'adiponitrile sur débit massique de catalyseur dans le réacteur piston, dans un intervalle donné pour un domaine d'activité du catalyseur déterminé d'obtenir simultanément une formation très limitée d'impuretés telles que par exemple, les diamines cycliques et une vitesse de dégradation de l'activité du catalyseur réduite.

Selon une caractéristique de l'invention, la réaction d'hydrogénation est réalisée en présence d'un solvant. Parmi les solvants convenables, l'amine obtenue par l'hydrogénation est le solvant préféré. Ainsi, dans le cas de l'hydrogénation de l'adiponitrile, l'hexaméthylène diamine est avantageusement utilisée comme composant principal du solvant. La concentration en amine dans le solvant est avantageusement comprise entre 50% et 99% en poids de préférence entre 60 et 99% en poids du solvant.

La réaction d'hydrogénation est réalisée, avantageusement, en présence d'eau comme autre composant du solvant. Cette eau est généralement présente dans une quantité inférieure ou égale à 50%, avantageusement inférieure ou égale à 20 % en poids par rapport au milieu réactionnel total, et encore plus préférentiellement entre 0,1 % et 15 % en poids.

En complément ou en substitution à l'eau, il est possible d'utiliser au moins un autre solvant, du type alcool. Comme alcools convenables, on peut citer par exemple, le méthanol, l'éthanol, le propanol, l'isopropanol, le butanol, les glycols comme l'éthylène glycol, des polyols ou un mélange de ces composés.

Le catalyseur d'hydrogénation comprend un métal de Raney de préférence du nickel de Raney ou du cobalt de Raney. Des éléments promoteurs peuvent être avantageusement utilisés avec le métal de Raney. Ces éléments promoteurs sont choisis dans les éléments appartenant aux groupes IIB, IVB à VIIB de la classification périodique des éléments. Avantageusement, les éléments promoteurs sont choisis dans le groupe comprenant le titane, le chrome, le zirconium, le vanadium, le molybdène, le manganèse, le cobalt, le nickel, le zinc, le fer et leurs associations.

La réaction d'hydrogénation est réalisée en présence d'un composé basique, de préférence une base minérale telle que LiOH, NaOH, KOH, RbOH, CsOH et leurs mélanges. NaOH et KOH sont préférentiellement utilisées.

La quantité de base ajoutée est déterminée pour avoir au moins 0,1 mole de base par kilogramme de catalyseur de préférence entre 0,1 et 2 moles de base par kg de catalyseur, et encore, plus avantageusement entre 0,3 et 1,5 moles de base par kg de catalyseur.

La réaction d'hydrogénation est réalisée à une température inférieure ou égale à 150°C, de préférence inférieure ou égale à 120°C et encore plus préférentiellement inférieure ou égale à 100°C. La température de la réaction est généralement comprise entre 50°C et 100 °C.

La pression en hydrogène dans le réacteur est comprise entre 0,10 et 10 MPa environ.

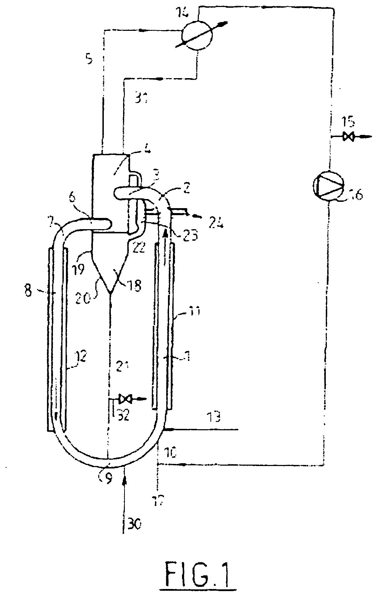

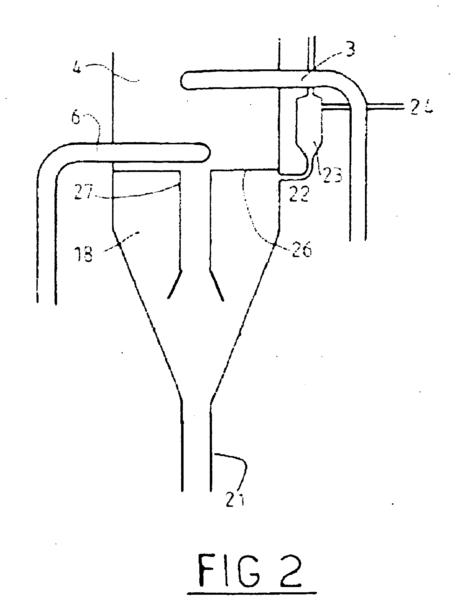

Selon un mode de réalisation préféré de l'invention la réaction d'hydrogénation de l'invention est mise en œuvre, en continu, dans un appareillage ou dispositif décrit ci-dessous en référence aux figures 1 et 2 annexées qui représentent des schémas synoptiques d'un mode de réalisation d'un appareillage convenable pour la mise en œuvre de l'invention.

L'appareillage convenant à la mise en oeuvre du procédé de l'invention permet de réaliser un excellent contact gaz/liquide, une séparation rapide et efficace de ces deux phases après contact, une séparation continue de l'hydrogénat et du catalyseur et le recyclage de ce dernier, en un temps compatible avec une désactivation la plus faible possible dudit catalyseur.

Ledit appareillage comporte trois sections principales : une section réaction fonctionnant selon le principe de la colonne à bulles avec circulation d'un lit de catalyseur en suspension, une section séparation gaz-liquide et une section séparation catalyseur-liquide avec recyclage dudit catalyseur et soutirage du liquide (hydrogénat).

La section réaction comporte généralement un ou plusieurs tubes en forme de U dont les branches sont verticales ou légèrement inclinées par rapport à la verticale, l'une des branches de chaque U assurant la montée de la dispersion gaz/liquide/catalyseur solide, l'autre le retour du liquide au moins partiellement dégazé. Elle comporte également quatre arrivées à la base de la branche montante : l'arrivée d'hydrogène, l'arrivée de l'adiponitrile, l'arrivée du catalyseur neuf, avec ou sans cocatalyseur et l'arrivée du catalyseur recyclé.

La section séparation gaz-liquide est constituée d'un cylindre vertical comportant une ou plusieurs arrivées tangentielles (venant de la branche montante du réacteur), un ou plusieurs départs tangentiels (vers la branche descendante du réacteur), une sortie de gaz et une sortie du mélange réactionnel vers la séparation liquide-solide. L'arrivée de la dispersion gaz/liquide/catalyseur solide s'effectue en un point situé au-dessus du point de départ du liquide dégazé. Cette partie forme une tête cyclone.

La section séparation liquide-solide est constituée d'un décanteur qui permet de séparer l'hydrogénat du catalyseur et de recycler ledit catalyseur. L'hydrogénat est soutiré en continu tandis que la suspension de catalyseur séparée dans le décanteur est ramenée dans la section réaction, en amont du point d'introduction de l'hydrogène. Une purge est réalisée lorsqu'il est jugé nécessaire de remplacer une partie du catalyseur par du catalyseur neuf.

Par catalyseur neuf, il faut comprendre dans le présent texte soit un catalyseur dit vierge c'est-à-dire n'ayant jamais été utilisé dans une réaction d'hydrogénation, soit un mélange de catalyseur vierge et de catalyseur régénéré ou encore uniquement du catalyseur régénéré. Le catalyseur neuf peut être introduit dans le réacteur après mélange du catalyseur vierge et régénéré ou sous forme de deux flux distincts de catalyseur vierge et catalyseur régénéré.

L'appareillage convenant au procédé de l'invention peut être illustré à titre d'exemple par la figure 1. Il comporte un tube vertical cylindrique (1) relié par un tube coudé (2) à un tube horizontal (3) débouchant tangentiellement dans un séparateur gaz/liquide (4) constitué par un cylindre vertical de diamètre supérieur à celui du tube (1).

Le séparateur (4) comporte une tubulure (5) d'évacuation de gaz ou de vapeurs. Un tube horizontal (6) prenant naissance tangentiellement au séparateur et en un point situé au-dessous du point d'arrivée du tube (3), est relié par l'intermédiaire d'un tube coudé (7) à un second tube vertical (8) communiquant avec le tube (1) par un coude (9). L'ensemble des tubes (1) et (8) et du coude (9) forme un U. Le tube (1) comporte à sa base une tubulure (10) d'introduction d'hydrogène et une tubulure (13) d'introduction de l'adiponitrile. Les tubes (1) et (8) peuvent comporter, comme représenté sur la figure 1, une double enveloppe (11) et (12) permettant la circulation d'un fluide de refroidissement ou de réchauffage. Le coude (9) comporte une arrivée (30) de catalyseur neuf et une arrivée (21) de catalyseur recyclé.

Les tubes (1) et (8) peuvent être verticaux ou légèrement obliques (dans ce dernier cas de préférence de telle sorte que leurs axes convergent vers le bas).

Les rayons de courbure des coudes (2, 7, 9) sont calculés selon les règles habituelles du génie chimique, de manière que la perte de charge de la masse en circulation dans l'ensemble du circuit soit aussi faible que possible. Leur angle de courbure peut varier de 45° à 135° et de préférence de 60° à 120°.

Sur la figure 1, l'hydrogène est introduit par une tubulure (10). Cette tubulure peut être munie de tout dispositif usuel de dispersion, mais un simple tube affleurant la paroi, disposé dans l'axe du tube (1) est suffisant. Cette tubulure (10) est reliée à une source d'hydrogène et celui-ci peut être introduit à la pression atmosphérique ou sous une pression supérieure.

La tubulure (5) d'évacuation des gaz peut être reliée à un dispositif quelconque de traitement des gaz séparés de l'hydrogénat. La figure 1 illustre un dispositif selon lequel les gaz issus de (5) passent dans un condenseur (14), dans lequel les vapeurs entraînées dans le séparateur (4) sont séparées de l'hydrogène. Le condensat obtenu est recyclé dans l'appareil par une tubulure (31). L'hydrogène en excès passe ensuite dans un compresseur (16) par une canalisation comportant un système de purge (15), puis il est recyclé en (10) après introduction en (17) d'une quantité d'hydrogène destinée à compenser l'hydrogène consommé au cours de l'hydrogénation et celui qui a été purgé.

Il est nécessaire de soutirer l'hydrogénat formé dégazé et débarrassé du catalyseur. Pour pouvoir soutirer un hydrogénat clair, c'est-à-dire ne comportant pratiquement pas de catalyseur, un décanteur (18) est placé directement sous le séparateur (4). La suspension liquide/catalyseur dont la phase gazeuse a été séparée dans le séparateur (4) pénètre dans le décanteur (18).

Le décanteur (18) est constitué par un cylindre (19) terminé par un cône (20).

Une canalisation (21) sert à ramener en continu dans le coude (9) la bouillie concentrée de catalyseur. L'hydrogénat débarrassé du catalyseur sort par une canalisation (22) reliée à un pot (23) muni d'un trop-plein (24) permettant de soutirer en continu l'hydrogénat clair, le niveau dans l'ensemble de l'appareil étant maintenu constant par l'introduction continue d'un volume équivalent de mélange adiponitrile, solvant et catalyseur. Le catalyseur décanté dans le cône (20) est recyclé dans le tube (9) par la tubulure (21). La tubulure (21) comporte une tubulure (32) de purge du catalyseur usé, qui pourra éventuellement être régénéré.

La figure 2 illustre plus en détail un mode particulier de décantation entrant dans le cadre de l'invention. Pour éviter, d'une part, que les mouvements trop rapides de la masse de catalyseur et d'hydrogénat ne se propagent dans le décanteur (18) et, d'autre part, que l'hydrogène ne pénètre dans ce dernier, une séparation entre les deux zones (section séparation gaz-liquide et section séparation liquide-solide) est nécessaire. Elle ne doit cependant en aucune façon être la cause d'un dépôt de catalyseur. Un tel résultat est obtenu grâce à la mise en place entre le séparateur (4) et le décanteur (18) d'une cloison (26), la circulation entre le séparateur gaz-liquide et le décanteur étant assurée par une canalisation (27) de diamètre calculé pour réduire notablement la vitesse du liquide (par exemple à une valeur inférieure à 0,5 mètre/seconde).

Cette canalisation (27) se prolonge à l'intérieur du décanteur par un tube de diamètre supérieur ou égal à celui de la canalisation (27).

L'utilisation du dispositif ou installation précédemment décrit pour la réalisation du procédé d'hydrogénation de l'adiponitrile en hexaméthylène diamine permet d'obtenir une bonne dispersion de l'hydrogène dans le mélange liquide réactionnel. Cette dispersion est stable et homogène dans tout le tube ou les tubes en U. Cet appareillage permet comme cela a été dit précédemment de séparer en continu et sans désactivation importante du catalyseur l'hydrogénat à soutirer dudit catalyseur à recycler.

Dans un tel appareillage, le rapport P, cité précédemment, entre le débit molaire de l'adiponitrile et le débit massique de catalyseur est celui existant dans la zone de réaction ou réacteur piston correspondant au tube (1). Toutefois, pour déterminer le débit massique de catalyseur, on déterminera avantageusement la concentration en catalyseur recyclé existante dans le tube (21) de recyclage du catalyseur ou la concentration en catalyseur dans le tube (8) de retour du milieu réactionnel.

D'autres détails, avantages de l'invention apparaîtront plus clairement au vu des exemples donnés ci-dessous à titre d'illustration uniquement

Exemple 1 :

Dans une installation représentée à la figure 1, on procède à l'hydrogénation de l'adiponitrile en hexaméthylène diamine en continu à une température de 82°C et une pression de 23 bar. Le milieu réactionnel contient de l'adiponitrile alimenté par le tube (13), de l'hydrogène alimenté par le tube (17), de la potasse, de l'hexaméthylène diamine, de l'eau et un catalyseur comprenant du nickel de Raney.
Le catalyseur présente une activité de $26.10^{-5}$ mole de $H_2$/g de catalyseur/seconde mesurée selon le test décrit ci-dessus. La concentration du catalyseur et le débit d'alimentation de l'adiponitrile sont réglés pour obtenir un facteur P dans le réacteur (1) d'hydrogénation compris entre 0.041 et 0.054 mole d'AdN / kg de catalyseur.

L'hexaméthylène diamine récupérée en (24) présente les caractéristiques de pureté suivantes (mesurées selon les méthodes décrites ci-dessus ou les techniques classiques) :

- concentration en diaminocyclohexane (DCH) : 1900 ppm
- Iuv : 1.5

Exemple 2 :

L'exemple 1 a été reproduit avec un catalyseur présentant une activité de $42.10^{-5}$ mole de $H_2$/g de catalyseur/seconde. La concentration du catalyseur et le débit d'AdN dans le réacteur (1) étant fixés pour obtenir un facteur P dans le réacteur compris entre 0,042 et 0,055 mole d'AdN / kg de catalyseur. L'hexaméthylène diamine récupérée en (24) présente les caractéristiques de pureté suivantes :

- concentration en diaminocyclohexane (DCH) : 3500 ppm
- Iuv : 5

Ces essais montrent clairement l'influence de l'activité du catalyseur pour un facteur P compris dans un certain domaine, sur la qualité de l'hexaméthylène diamine produite en sortie de réacteur.

**Revendications**

1. Procédé continu de synthèse de composés comprenant au moins une fonction amine par hydrogénation d'un composé comprenant au moins une fonction nitrile **caractérisé en ce qu'**il consiste à :

   ❖ Alimenter un gaz contenant de l'hydrogène et un composé nitrile, dans un réacteur de type piston dans lequel circule un milieu réactionnel comprenant des particules en suspension de catalyseur à base de métal de Raney, une base minérale et de l'eau,
   ❖ soutirer en sortie du réacteur piston, une partie du milieu réactionnel contenant le composé amine après séparation du catalyseur et recycler l'autre partie dans le réacteur piston.
   ❖ recycler le catalyseur séparé dans le réacteur piston,
   ❖ alimenter dans le réacteur piston un flux de catalyseur neuf,

   **en ce que**, les débits d'alimentation en composés nitriles et/ou en catalyseur dans le réacteur piston sont contrôlés pour maintenir un rapport P entre le nombre de mole de composés nitriles alimentés par unité de temps et le débit massique de catalyseur dans le réacteur piston, compris entre 0,02 et 0,15 mole de composés nitriles par kg de catalyseur pour un catalyseur présentant une activité initiale comprise entre $15.10^{-5}$ et $35.10^{-5}$ mole de $H_2$/g de catalyseur /s,
   et **en ce que** le composé nitrile est l'adiponitrile, l'aminé synthétisée étant l'hexaméthylène diamine.

2. Procédé selon la revendication 1, **caractérisé en ce que** le milieu réactionnel comprend un solvant.

3. Procédé selon la revendication 2, **caractérisé en ce que** le solvant est l'aminé produite par la réaction d'hydrogénation.

4. Procédé selon l'une des revendications précédentes, **caractérisé en ce qu'**il comprend, en sortie de réacteur piston, une zone de décantation des particules de catalyseur, la phase surnageante étant recyclée dans le réacteur piston par une première boucle externe comportant un prélèvement du milieu contenant l'aminé, la phase décantée étant recyclée dans le réacteur piston par une seconde boucle externe.

5. Procédé selon la revendication 4, **caractérisé en ce qu'**il comprend un soutirage de catalyseur usé à partir de la seconde boucle externe, et une alimentation en catalyseur neuf à l'entrée du réacteur piston ou dans la seconde boucle externe.

**6.** Procédé selon la revendication 5, **caractérisé en ce que** le catalyseur neuf est constitué de catalyseur vierge, catalyseur régénéré ou un mélange de ceux-ci.

**7.** Procédé selon l'une des revendications 4 à 6, **caractérisé en ce qu'**il comprend une détermination de la concentration en catalyseur dans la seconde boucle extérieure pour calculer la masse totale de catalyseur dans le réacteur piston.

**8.** Procédé selon l'une des revendications précédentes, **caractérisé en ce que** le catalyseur est à base de nickel de Raney ou cobalt de Raney.

**9.** Procédé selon la revendication 8, **caractérisé en ce que** le catalyseur comprend un promoteur choisi dans les éléments appartenant aux groupe IIB, IVB à VIIB de la classification périodique des éléments et leurs associations.

**10.** Procédé selon l'une des revendications précédentes, **caractérisé en ce que** la base minérale est choisie dans le groupe comprenant LiOH, NaOH, KOH, RbOH, CsOH ou leurs mélanges.

**11.** Procédé selon la revendication 10, **caractérisé en ce que** la base minérale est choisie dans le groupe comprenant KOH et NaOH ou leurs mélanges.

**12.** Procédé selon la revendication 10 ou 11, **caractérisé en ce que** la quantité de base dans le milieu réactionnel est comprise entre 0,1 mole de base par kg de catalyseur et 2 moles de base par kg de catalyseur.

**13.** Procédé selon l'une des revendications précédentes, **caractérisé en ce que** la température de la réaction est comprise entre 50°C et 150°C et la pression en hydrogène est comprise entre 0.1MPa et 10MPa.

**Patentansprüche**

**1.** Kontinuierliches Verfahren zur Synthese von Verbindungen mit mindestens einer Aminfunktion durch Hydrierung einer Verbindung mit mindestens einer Nitrilfunktion, **dadurch gekennzeichnet, dass** es aus Folgendem besteht:

❖ Einspeisen eines Wasserstoff und eine Nitrilverbindung enthaltenden Gases in einen Reaktor vom Kolbenströmungstyp, in dem ein Reaktionsmedium, das suspendierte Teilchen eines Katalysators auf Basis von Raney-Metall, eine anorganische Base und Wasser umfasst, strömt,
❖ Abziehen eines Teils des die Aminverbindung enthaltenden Reaktionsmediums am Ausgang des Kolbenströmungsreaktors nach Abtrennung des Katalysators und Rückführung des anderen Teils in den Kolbenströmungsreaktor,
❖ Rückführen des abgetrennten Katalysators in den Kolbenströmungsreaktor,
❖ Einspeisen eines Stroms von neuem Katalysator in den Kolbenströmungsreaktor,

und dadurch, dass die Durchsätze der Einspeisung von Nitrilverbindungen und/oder Katalysator in den Kolbenströmungsreaktor so kontrolliert werden, dass für einen Katalysator mit einer Anfangsaktivität zwischen $15.10^{-5}$ und $35.10^{-5}$ mol $H_2$/g Katalysator/s ein Verhältnis P zwischen der Zahl der Mole pro Zeiteinheit eingespeister Nitrilverbindungen und des Massendurchsatzes des Katalysators in dem Kolbenströmungsreaktor zwischen 0,02 und 0,15 mol Nitrilverbindungen pro kg Katalysator gehalten wird, und **dadurch gekennzeichnet, dass** es sich bei der Nitrilverbindung um Adiponitril handelt, wobei es sich bei dem synthetisierten Amin um Hexamethylendiamin handelt.

**2.** Verfahren nach Anspruch 1, **dadurch gekennzeichnet, dass** das Reaktionsmedium ein Lösungsmittel umfasst.

**3.** Verfahren nach Anspruch 2, **dadurch gekennzeichnet, dass** es sich bei dem Lösungsmittel um das durch die Hydrierungsreaktion gebildete Amin handelt.

**4.** Verfahren nach einem der vorhergehenden Ansprüche, **dadurch gekennzeichnet, dass** es am Ausgang des Kolbenströmungsreaktors eine Zone zum Absetzenlassen der Katalysatorteilchen umfasst, wobei die überstehende Phase über eine erste externe Schlaufe, die einen Austrag des das Amin enthaltenden Mediums umfasst, in den Kolbenströmungsreaktor zurückgeführt wird und die abgesetzte Phase über eine zweite externe Schlaufe in den Kolbenströmungsreaktor zurückgeführt wird.

**5.** Verfahren nach Anspruch 4, **dadurch gekennzeichnet, dass** es das Abziehen von verbrauchtem Katalysator aus der zweiten externen Schlaufe und das Einspeisen von neuem Katalysator am Eingang des Kolbenströmungsreaktors oder in die zweite externe Schlaufe umfasst.

**6.** Verfahren nach Anspruch 5, **dadurch gekennzeichnet, dass** der neue Katalysator aus frischem Katalysator, regeneriertem Katalysator oder einer Mischung davon besteht.

**7.** Verfahren nach einem der Ansprüche 4 bis 6, **da-**

**durch gekennzeichnet, dass** es das Bestimmen der Katalysatorkonzentration in der zweiten externen Schlaufe zur Berechnung der Katalysatorgesamtmasse in dem Kolbenströmungsreaktor umfasst.

8. Verfahren nach einem der vorhergehenden Ansprüche, **dadurch gekennzeichnet, dass** der Katalysator auf Raney-Nickel oder Raney-Cobalt basiert.

9. Verfahren nach Anspruch 8, **dadurch gekennzeichnet, dass** der Katalysator einen unter den Elementen der Gruppe IIB und IVB bis VIIB des Periodensystems der Elemente und Kombinationen davon ausgewählten Promotor umfasst.

10. Verfahren nach einem der vorhergehenden Ansprüche, **dadurch gekennzeichnet, dass** die anorganische Base aus der Gruppe bestehend aus LiOH, NaOH, KOH, RbOH, CsOH oder Mischungen davon ausgewählt wird.

11. Verfahren nach Anspruch 10, **dadurch gekennzeichnet, dass** die anorganische Base aus der Gruppe bestehend aus KOH und NaOH oder Mischungen davon ausgewählt wird.

12. Verfahren nach Anspruch 10 oder 11, **dadurch gekennzeichnet, dass** die Basenmenge in dem Reaktionsmedium zwischen 0,1 mol Base pro kg Katalysator und 2 mol Base pro kg Katalysator liegt.

13. Verfahren nach einem der vorhergehenden Ansprüche, **dadurch gekennzeichnet, dass** die Reaktionstemperatur zwischen 50°C und 150°C liegt und der Wasserstoffdruck zwischen 0,1 MPa und 10 MPa liegt.

## Claims

1. Continuous process for the synthesis of compounds comprising at least one amine functional group by hydrogenation of a compound comprising at least one nitrile functional group, **characterized in that** it consists in:

   ❖ feeding a gas comprising hydrogen and a nitrile compound to a reactor of plug-flow type in which circulates a reaction medium comprising suspended particles of catalyst based on Raney metal, an inorganic base and water,
   ❖ withdrawing, at the outlet of the plug-flow reactor, a portion of the reaction medium comprising the amine compound after separation of the catalyst and recycling the other portion to the plug-flow reactor,
   ❖ recycling the separated catalyst to the plug-flow reactor,
   ❖ feeding a stream of fresh catalyst to the plug-flow reactor,

   and **in that** the flow rates for feeding nitrile compounds and/or catalyst to the plug-flow reactor are controlled in order to maintain a ratio P of the number of moles of nitrile compounds fed per unit of time to the flow rate by weight of catalyst in the plug-flow reactor of between 0.02 and 0.15 mol of nitrile compounds per kg of catalyst for a catalyst exhibiting an initial activity of between $15 \times 10^{-5}$ and $35 \times 10^{-5}$ mol of $H_2$/g of catalyst/s, and **in that** the nitrile compound is adiponitrile, the amine synthesized being hexamethylenediamine.

2. Process according to Claim 1, **characterized in that** the reaction medium comprises a solvent.

3. Process according to Claim 2, **characterized in that** the solvent is the amine produced by the hydrogenation reaction.

4. Process according to one of the preceding claims, **characterized in that** it comprises, at the outlet of the plug-flow reactor, a region for decantation of the catalyst particles, the supernatant phase being recycled to the plug-flow reactor via a first external loop comprising a withdrawal of the medium comprising the amine, the decanted phase being recycled to the plug-flow reactor via a second external loop.

5. Process according to Claim 4, **characterized in that** it comprises withdrawing spent catalyst from the second external loop and feeding fresh catalyst at the inlet of the plug-flow reactor or to the second external loop.

6. Process according to Claim 5, **characterized in that** the fresh catalyst is composed of virgin catalyst, regenerated catalyst or a mixture of these.

7. Process according to one of Claims 4 to 6, **characterized in that** it comprises a determination of the concentration of catalyst in the second external loop in order to calculate the total weight of catalyst in the plug-flow reactor.

8. Process according to one of the preceding claims, **characterized in that** the catalyst is based on Raney nickel or Raney cobalt.

9. Process according to Claim 8, **characterized in that** the catalyst comprises a promoter chosen from the elements belonging to Groups IIB and IVB to VIIB of the Periodic Table of the Elements and their combinations.

**10.** Process according to one of the preceding claims, **characterized in that** the inorganic base is chosen from the group consisting of LiOH, NaOH, KOH, RbOH, CsOH and their mixtures.

**11.** Process according to Claim 10, **characterized in that** the inorganic base is chosen from the group consisting of KOH and NaOH and their mixtures.

**12.** Process according to Claim 10 or 11, **characterized in that** the amount of base in the reaction medium is between 0.1 mol of base per kg of catalyst and 2 mol of base per kg of catalyst.

**13.** Process according to one of the preceding claims, **characterized in that** the reaction temperature is between 50°C and 150°C and the hydrogen pressure is between 0.1 MPa and 10 MPa.

FIG.1

FIG 2

**EP 2 064 171 B2**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 0037424 A

**Littérature non-brevet citée dans la description**

- *Chemical Engineering Science,* 1992, vol. 47 (9-11), 289-94
- *Chemical Engineering Science,* 1980, vol. 35, 135-141

13